Europäisches Patentamt

European Patent Office

Office européen des brevets

(19)

(11) **EP 1 089 695 B1**

(12) **EUROPEAN PATENT SPECIFICATION**

(45) Date of publication and mention
of the grant of the patent:
**09.04.2003 Bulletin 2003/15**

(51) Int Cl.⁷: **A61F 13/15**, A61F 5/451

(21) Application number: **99928523.2**

(22) Date of filing: **11.06.1999**

(86) International application number:
**PCT/US99/13002**

(87) International publication number:
**WO 00/000134 (06.01.2000 Gazette 2000/01)**

(54) **FAECAL COLLECTOR HAVING BREATHABLE SKIN ATTACHMENT MEANS**

FÄKALIENAUFNAHMEBEHÄLTER MIT EINE ATMUNGSAKTIVE LASCHE ZUR BEFESTIGUNG AN DER HAUT

COLLECTEUR DE MATIERES FECALES DOTE D'UN DISPOSITIF RESPIRANT D'ATTACHE SUR LA PEAU

(84) Designated Contracting States:
**DE ES FR GB IT**

(30) Priority: **26.06.1998 WOPCT/US98/13291**

(43) Date of publication of application:
**11.04.2001 Bulletin 2001/15**

(73) Proprietor: **THE PROCTER & GAMBLE COMPANY**
**Cincinnati, Ohio 45202 (US)**

(72) Inventors:
• **PALUMBO, Gianfranco**
**D-65760 Eschborn (DE)**
• **D'ACCHIOLI, Vincenzo**
**D-65779 Kelkheim am Taunus (DE)**

(74) Representative: **Veronese, Pancrazio et al**
**Procter & Gamble Italia S.p.A.**
**Italian Research Center**
**Via Aterno 92/94**
**66020 Sambuceto di San Giovanni Teatino**
**(Chieti) (IT)**

(56) References cited:
**EP-A- 0 753 290        GB-A- 1 078 588**
**GB-A- 1 092 274        GB-A- 2 116 849**
**US-A- 3 577 989**

• **PATENT ABSTRACTS OF JAPAN vol. 096, no. 009, 30 September 1996 (1996-09-30) & JP 08 117261 A (BEPPU YOSHIO), 14 May 1996 (1996-05-14)**

**Description**

Field of the Invention

The present invention relates to a disposable human waste management devices such as urine management devices and faecal management device having a skin attachment device which has a softer tactile feel for increased wearer comfort and satisfaction which is breathable.

Background of the Invention

Urine and faecal management devices are known articles of manufacture that are designed to be worn principally by incontinence sufferers and in particular by bedridden patients. Such faecal management devices are attached to the natural anal region or artificial anus of the wearer and or the uro genital region and are intended to entrap and immediately contain faecal material and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves.
Such devices as they are mostly known today are designed to be worn by bedridden patients. As such the devices are constituted of a relatively long and narrow tube, at one extremity of which there is an aperture surrounded by a flange upon which an adhesive can be applied in order to attach the device to the wearer. The flanges are typically comprised of flexible material which can stretch and contract so that the aperture size can be increased for example upon defecation. The materials typically utilised are closed cell foams.
Such flanges are disclosed for example in US 3,577,989, which details a disposable elimination-trapping bag for incontinence sufferers including a container member having an open-top portion, and an expandable flange secured to the container member around the open-top portion. The flange may include a layer of adhesive on its surface as a means of attachment of the bag to the wearer or alternatively discloses the use of elastic straps to attach the bag to the wearer. US 4,784,656 also describes a receptacle for collecting faecal matter from incontinence sufferers. The faecal collector comprises a gasket, a conduit means or a cylinder and a receptacle; the gasket is made of soft pliable stretchable material such as a closed cell foam and the side surface of the gasket is coated with a layer of adhesive; GB 2 152 387, teaches a faecal collector for incontinence sufferers comprising a collection bag and a ring, which is provided with an adhesive. The ring can extend and contract and is a closed cell thermoplastic foam.
Patent applications EP 753290 and GB 2116849 also disclose a fecal management bag having a patch for attachment to the skin provided with an adhesive.
Due to their typical elongated shape and dimensions, such devices particularly when worn by active wearers, such as infants or non bedridden incontinent adults, can readily twist around the thighs of the wearers and/or can cause the formation of folds and kinks in the devices themselves. Under such circumstances the pressure and stress exerted upon the bag and in particular the flange will naturally increase due to the movement of the wearer and the pressure of the wearer's body upon the bag. Consequently, the likelihood that the excreted material once excreted and contained within the bag will be caused to exert pressure upon the attachment means of the device will increase and as a result not only will the storage capacity of the device be detrimentally affected, but also more importantly, it may result in unintentional detachment of the device from the wearer during use. Such an occurrence is unacceptable causing distressing consequences for both the wearer and the carer.
Hence, it is critical that the human waste management devices are designed such that they securely attached to the skin of the wearer and do not become unintentionally unattached during all circumstances of use.
In order to provide the desired level of adhesion of the device to the wearer, the prior art typically discloses the utilisation of certain adhesives having very high cohesive strengths such as rubber based adhesives, acrylics and hydrocolloid adhesives as for example described in EP 245 064. These adhesives are then applied as thick layers over the entire surface of the flange of the device to maximise the adhesive force by which the device is secured to the skin of the wearer. Indeed it is apparent that for these devices and in particular the adhesives that have been designed for use on faecal management devices utilised by bedridden patients, particularly those having an artificial anus, maximum adhesion takes priority over any other criteria such as skin compatibility and patient comfort.
In particular these skin attachment devices raise a number of problems related to the comfort of the wearer of such devices. Whilst the skin attachment means are satisfactory in terms of adhesion to the skin of the wearer, on the other hand they do not allow for easy removal of the device from the wearer, without the wearer experiencing unacceptable pain levels. However providing a painless adhesive is essential in order to allow for the application of such devices on sensitive skin and wearer groups such as infants and is especially important under circumstances, where the device is misplaced, and removal and reapplication of the device once or even a number of times is required. Another problem related to the utilisation of such adhesives is that use of such can lead to reddening, skin rashes and perhaps, even lead to a more severe skin irritation. Moreover another problem related to the use of adhesives is that

**EP 1 089 695 B1**

the skin is entirely occluded over the are of application, causing hot and clammy conditions and skin stickiness in use. Such problems which are even further exacerbated when the device is used in combination with a diaper may cause such discomfort that the device is ultimately disregarded. Therefore, a real consumer need can be identified for a human waste management device with skin compatibility benefits.

However none of the previously mentioned prior art in the field of faecal management bags even recognises or addresses the problem of providing these devices with skin attachment means which meet these criteria.

Hence there still exists a need to provide a human waste management device having a skin attachment means which is compliant and flexible whilst ensuring the secure attachment and painless removal of the device from the skin in-between the buttocks of the wearer so as to be suitable for use of sensitive skin of an infant. It is thus an object of the present invention to provide such as device.

It has now been found that the above drawbacks will be substantially alleviated by providing the skin attachment means for use in human waste management devices comprising a flange applied with a suitable adhesive wherein the attachment means is breathable i.e. is liquid impermeable and moisture vapour permeable. In this manner the skin is able to function normally despite being in contact with the attachment means and can for example perspire normally at elevated temperatures such as those arising in the occlusive environment between the skin and the device and thereby ensure that the skin is cooled.

In another aspect of the present invention, the faecal management device with its breathable skin attachment device can be advantageously used with a disposable diaper.

US 4 699 146 does disclose water insoluble hydrophilic pressure sensitive adhesives which are comfortable, soft and elastomeric. The adhesives are formed from cross linked organic polymers of polyvinylpyrolidine with plasticizers and find application in a number of articles such as bandages and wound dressing as well as ostomy devices. According to this document the adhesive may have absorb moisture vapour. However whilst this document recognises the importance of providing a comfortable adhesive providing multiple benefits, the skin attachment means which are capable of moisture vapour transport are not disclosed.

Summary of the Invention

The present invention relates to human waste management devises such as urine management devices and faecal management device (10) comprising a bag (11), said bag (11) having an aperture and a skin attachment means surrounding said aperture (21). The skin attachment means comprises a flange (12) having a wearer facing surface (23) and a garment facing surface (22), wherein said wearer facing surface (23) of said flange (12) comprises an adhesive (20) characterised in that said skin attachment means is liquid moisture vapour permeable and has a moisture vapour transport rate of at least 250g/(m$^2$.24hrs.).

Brief description of the drawings

It is believed that the invention will be better understood from the foregoing description in conjunction with the accompanying drawings in which:

Figure 1 is a perspective view of a faecal management device according to the present invention;

Figure 2 shows a perspective view of the faecal management device of the present invention in conjunction with a disposable diaper; and

Figure 3 is a partially cut-away perspective view of a disposable diaper embodying the present invention.

Figure 4 is a plan view of a disposable urine management device of the present invention.

Detailed Description of the Invention

Typically human waste management devices such as faecal management devices comprise a bag (11), said bag (11) having an aperture (21) and a skin attachment means surrounding said aperture (21). The skin attachment means comprises a flange (12) having a wearer facing surface (23) and a garment facing surface (22), wherein said wearer facing surface (23) of said flange (12) comprises an adhesive for attachment of the device to the perianal area and or uro genital area of a wearer.

In order to avoid the problem of entrapment and condensation of moisture vapour given off by the body of the wearer and thus, the hot, clammy and uncomfortable conditions after a short period of use the skin attachment means of the devices of the present invention are breathable i.e. moisture vapour permeable.

According to the present invention the skin attachment means of the human waste management device hence has a vapour permeability of greater than 250 g/(m$^2$.24hrs.), preferably greater than 300g/m$^2$/24hrs., more preferably greater than 500g/m$^2$/24hrs., most preferably greater than 600g/m$^2$/24hrs. In a preferred embodiment of the present invention the wall of the bag also has an air permeability of greater than 50 l/m$^2$/s, more preferably greater than 60l/m$^2$/s, most preferably greater than 70l/m$^2$/s.

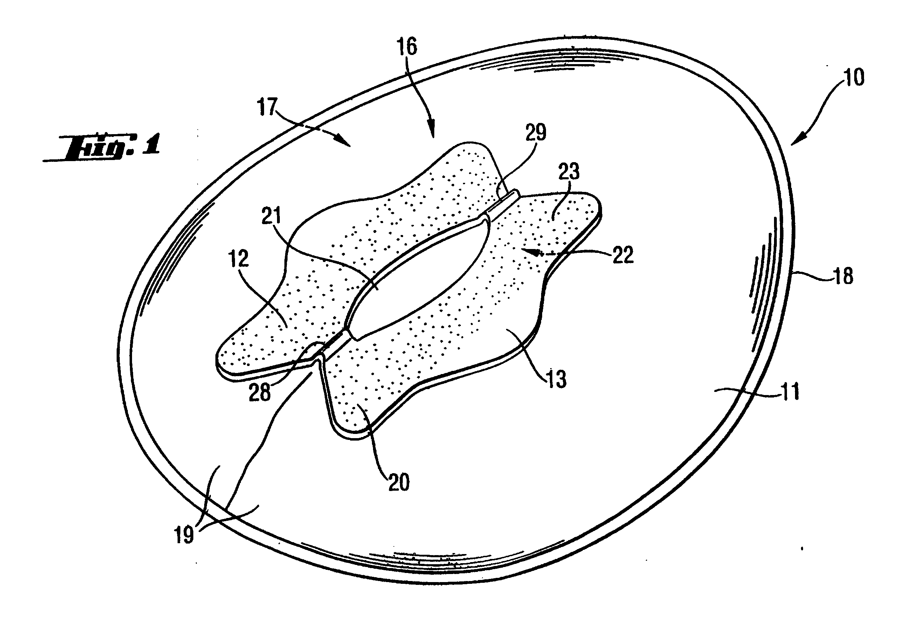

As shown in Figure 1 the bag (11) is provided with an aperture (21) whereby faecal matter is received from the body prior to storage within the bag cavity. The aperture (21) is surrounded a skin attachment means. The skin attachment means comprises a flange (12) which may be provided in any shape or size, such as circular, oblong, heart shaped and may be symmetrical or asymmetrical, preferably the aperture has an oblong configuration either in the longitudinal or in the transversal direction, most preferably the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering matter.

The flange may be provided in any size depending on the wearer group for which the device is intended. Similarly the flange may be provided in any shape and preferably has a symmetrical shape preferably comprising a plurality of lobes (13).

The flange comprises a garment facing surface (22) and a wearer facing surface (23). In an preferred embodiment these are two large, substantially flat surfaces, however, the flange may also comprise projections (28, 29) designed to fit the perineal or coccygeal area of the wearer.

The flange (12) should be made of soft, flexible and malleable material to allow easy placement of the flange to the perianal area. Any materials which meet the moisture vapour permeability requirements may be usefully employed herein Typical materials include nonwoven materials, wovens, open celled thermoplastic foams, composites of open celled foams and stretch nonwoven, and films and any combination thereof. Preferably, such foams have a thickness within the general range of 0.1 to 5 millimetres and a basis weight of 5 to 250 g/m$^2$, more preferably 50 g/m$^2$. Other thermoplastic foam materials, or other suitable plastics sheet materials having the described properties of such foams (i.e., moisture vapour permeability, softness, pliability, stretchability, and contractability) might also be used such as monolithic films, microporous and macroporous films and apertured formed films. Preferably the flange comprises an open celled foam, a nonwoven or a combination thereof. Preferably, the material of garment facing surface of the flange may extend into the defined aperture area so as to form a skirt or flap of material which prevents unintentional adhesion of the surface edges of the flange defining the aperture to oneanother during use.

According to the present invention the skin attachment means further comprises an adhesive applied to the wearer facing surface (23) of the flange (12) to secure the device to the wearer. The adhesive (20) is preferably covered with a release means (not shown) in order to protect the adhesive layer, such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 1, the adhesive is in one preferred embodiment not applied to the entire wearer facing surface area of the flange (12), so as to provide lobes (13) on either side of the flange (12) which are non-adhesive and can thereby serve to facilitate placement and removal of the device whilst avoiding contact with the adhesive. These lobes are however preferably also covered by the release paper. Before application of the human waste management device (10) to the skin of the wearer, the release means if present is removed.

According to the present invention any medically approved, water resistant, moisture vapour permeable, pressure sensitive adhesives comprising a polymer which forms a 3-dimensional matrix, and comprising less than 10%, preferably less than 5% by weight of said adhesive of hydrocolloids, may be used to attach the device to the perianal and or uro genital area of the wearer.

The term hydrocolloid as used herein refers to colloidal absorbent materials and mixtures of colloidal absorbent materials selected from starch, modified starches such as dextrin, cellulose ester such as carboxymethycellulose, natural gums such as pectin karaya, gelatin, guar gum, gum arabic, locust bean gum, and carboxypolymethylene.

According to the present invention the 3-dimensional matrix also referred to herein as a gel, comprises as an essential component a polymer which can be physically or chemically cross linked. The polymer may be naturally or synthetically derived. The uncrosslinked polymer includes repeating units derived from vinyl alcohols, vinyl ethers and their copolymers, carboxy vinyl monomer, vinyl ester monomers, esters of carboxy vinyl monomers, vinyl amide monomers, hydroxy vinyl monomers, cationic vinyl monomers containing amines or quaternary groups, N-vinyl lactam monomer, polyethylene oxides, polyvinylpyrrolidon (PVP), acrylics such as hydroxyethylmethacrylate, methoxydiethoxyethyl methacrylate and hydroxydiethoxyethyl methacrylate and sulphonated polymers such as acrylamide sulphonated polymers and mixtures thereof. Alternatively, the uncrosslinked polymer may be a homopolymer or copolymer of a polyvinyl ether, or a copolymer derived from half ester of maleic ester. Similarly any other compatible polymer monomer units may be used as copolymers such as for example polyvinyl alcohol and polyacrylic acid or ethylene and vinyl acetate.

Particularly preferred polymers are acrylics, sulphonated polymers such as acrylamide sulphonated polymers, vinyl alcohols, vinyl pyrrolidine, polyethylene oxide and mixtures thereof.

According to the present invention the 3 dimensional adhesive matrix also essentially comprises a plasticiser, which is preferably a liquid at room temperature. This material is selected such that the polymer may be solubilized or dispersed within the plasticiser. For embodiments wherein irradiation cross linking is to be carried out, the plasticiser must also be irradiation cross linking compatible such that it does not inhibit the irradiation cross linking process of the polymer. The plasticiser may be hydrophilic or hydrophobic.

Suitable plasticisers include water, alcohols, polyhydric alcohols such as glycerol and sorbitol, and glycols and ether glycols such as mono- or diethers of polyalkylene gylcol, mono- or diester polyalkylene glycols, polyethylene glycols (typically up to a molecular weight of about 600), glycolates, glyceril, sorbitan esters, esters of citric and tartaric acid, imidazoline derived amphoteric surfactants, lactams, amides, polyamides, quaternary ammonium compounds, condensation products of polyethylene imine and epichlorohydrin, liquid polybutenes, esters such phthalates, adipates, stearates, palmitates, sebacates, or myristates and combinations thereof. Particularly preferred are polyhydric alcohols, polyethylene glycol (with a molecular weight up to about 600), glycerol, sorbitol, water and mixtures thereof.

Typically the adhesive comprises a ratio of polymer to plasticiser by weight of from 1:100 to 100:1, more preferably from 50:1 to 1:50. However, the exact amounts and ratios of the polymer and plasticiser will depend to a large extent on the exact nature of polymer and plasticisers utilised and can be readily selected by the skilled person in the art. For example a high molecular weight polymer material will require a greater amount of plasticiser than a low molecular weight polymer.

In addition to the polymer and plasticiser components of the adhesive, the adhesive may comprise a number of optional additional components for example the composition may comprise from 0% to 50% by weight of the composition, of a tackifying resin. Such tackifying resins are particularly useful in combination with ABA block copolymer adhesive compositions. Suitable tackifying resins include for example rosin derivatives, terpene, and terpene-phenolic resins, hydrocarbon resins such as $C_5$ and $C_5/C_9$ resins, aromatic resins and hydrogenated resins.

Other suitable optional ingredients include from 0% to 10% and more preferably form 0% to 5% by weight of substances for further facilitating and stabilising the 3-dimensional matrix and the matrix forming process. For example for hydrophobic adhesive compositions these may be fatty acids of $C_8$ to $C_{22}$, their metallic salts and their polyoxo-derivatives; lanolin derivatives; silica; bentonite, montmorillonite and their derivatives; waxes or mixtures thereof.

Other common additives known in the art such as preservatives, antioxidants, anti UV agents, pigments, mineral fillers and mixtures thereof may also be comprised within the adhesive composition in quantities up to 10% each respectively.

According to the present invention the polymer component of the adhesive can be physically or chemically cross linked in order to form the 3-dimensional matrix. Physical cross linking refers to polymers having cross links which are not chemical covalent bonds but are of a physical nature such that there are areas in the 3 dimensional matrix having high crystallinity or areas having a high glass transition temperature. Chemical cross linking refers to polymers which are linked by chemical bonds. Preferably the polymer is chemically cross linked by radiation techniques such as thermal-, E beam-, UV-, gamma or micro-wave radiation.

In addition when chemical crosslinks are formed in the system, a polyfunctional crosslinker andlor a free radical initiator may be present in the premix to initiate the cross linking upon irradiation. Such an initiator can be present preferably in quantities up to 5% by weight

The resulting adhesive compositions suitable for use herein may be divided into two family types; hydrophilic and hydrophilic-hydrophobic mixed phase compositions dependant upon the nature of the components of the adhesive.

Hydrophilic adhesives are compositions in which typically the plasticiser is water or glycerol or glycol and/or mixtures thereof and the polymeric phase is of synthetic (e.g. polyacryltcs). Optionally such compositions may comprise up to 10% by weight of colloidal natural gums.

Hydrophobic adhesives are compositions in which the plasticiser is typically an oil or blend of oils of vegetable or mineral origin and the polymer is usually a synthetic polymer, preferably an elastomer, which is soluble or dispersible in such oils. Thus mixed phase adhesives are compositions in which both hydrophobic and hydrophilic components, possibly in both plasticisers and polymers, form two or more separate phases. In such cases an emulsifier/surfactant is preferably present at a suitable level to form stable emulsions between the incompatible phases.

The preferred adhesive compositions for use in the present invention are hydrophilic.

Suitable adhesives for use herein include Promeon, available from Promeon Division of Medtronic Inc., Minneapolis Minnesota, USA and hydrogel adhesive available from 3M.

The adhesive (20) can be applied to the wearer facing surface of the flange (12) by any means known in the art such as slot coating, spiral, or bead application or printing. Typically the adhesive is applied at a basis weight of from 20g/m² to 2500g/m², more preferably from 500g/m² to 2000g/m² most preferably from 700g/m² to 1500g/m² depending on the end use envisioned. For example for faecal management devices to be used for babies the amount of adhesive may be less than for faecal management devices designed for active adult incontinence sufferers.

The bag (11) as used herein is a flexible receptacle for the containment of excreted matter. The bag (11) can be provided in any shape or size depending on the intended use thereof, i.e. whether the device is intended for bedridden patients or active patients suffering from incontinence or requiring an artificial bowel or for infants. For example elongated bags which are principally tubular or rectangular are typically utilised by bedridden patients and elderly incontinence sufferers. For more active wearers whether infants or adults, the faecal management device should preferably be anatomically shaped such that the device follows the contours of the body and can be worn inconspicuously by the wearer under normal garments.

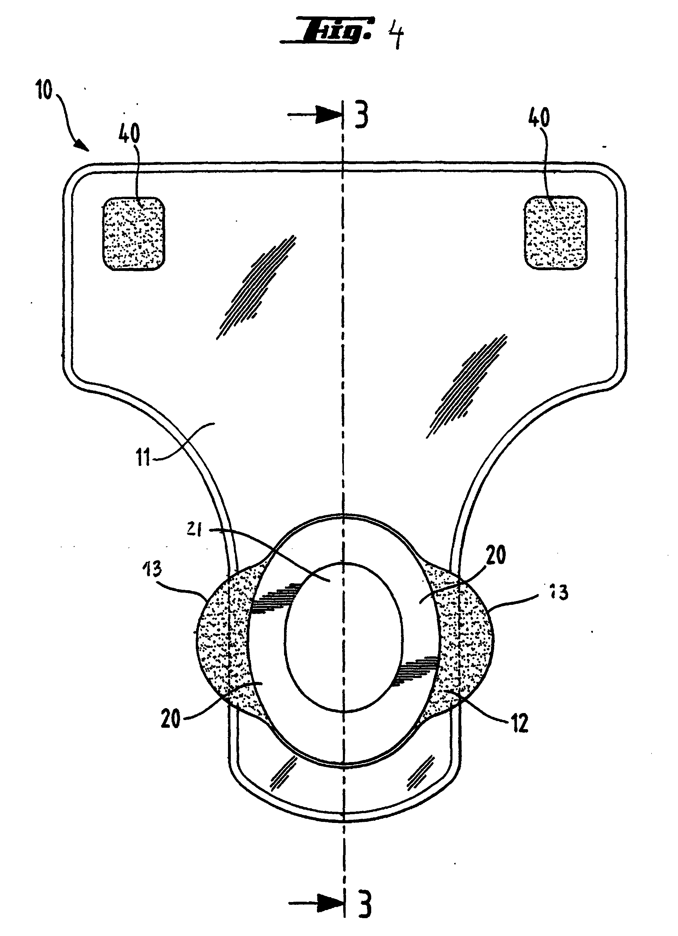

Particularly, preferred shapes are flat circular type bags, cone shaped bags, truncated shaped bags and pyramidal or truncated pyramidal or cone shaped bags. In a most preferred embodiment of a faecal management device of the present invention, the bag (11) has a substantially truncated cone shape. A preferred shape for urine bags is shown in figure 4. Typically the bags will have a wearer facing portion (16) and a garment facing portion (17). The wearer facing portion (16) of the faecal management device (10) is disposed adjacent the buttocks of the wearer. As such, the wearer facing portion (16) amply covers the buttocks of the wearer and does not hang between the thighs of the wearer.

In addition, the bag (11) is preferably shaped to allow at least partial insertion and retention of the bag in-between the buttocks of the wearer and thereby ensure good contact between the flange and the skin of the wearer. For example the faecal management bag may be provided with a neck portion or conduit.

The bag (11) is preferably designed to provide sufficient volume for excreted material under a variety of wearing conditions, also when worn by a freely moving, i.e. not bedridden wearer. Sitting on the bag, for example, will result in a largely reduced volume in some areas of the bag. Thus, the bag is preferably shaped to provide sufficient volume in areas which are not subjected to much pressure in wearing conditions such as sitting.

The bag (11) is designed to safely contain any entrapped material, typically it will be liquid impermeable, yet it may be breathable. The bag is designed of sufficient strength to withstand rupture in use, also when pressure on the bag is exerted in typical wearing conditions, such as sitting.

According to the present invention, depending on the shape of the bag (11) required, the bag (11) may be provided from a unitary piece of material or from a number of separate pieces of material, which may be identical or different and which are sealed at their respective peripheries.

In one preferred embodiment the bags herein have a wearer facingportion (16) and a garment facing portion (17) which comprise separate pieces of material. The wearer facing portion (16) and the -garment facing portion (17) are sealed at the periphery of the bag (11), thus creating a bag peripheral rim (18). As is visible from Figure 1, the wearer facing portion (16) of the bag (11) may comprise two further sections (19), which are secured to each other by means known to the man skilled in the art, such as adhesive, thermobonding or pressure bonding in order to provide the desired bag configuration.

According to the present invention the bag (11) can comprise one or multiple layers, preferably two or three layers. The layer on the inside of the bag, which will typically at least partially come in contact with excreted material is called the inner layer. The outermost layer of the bag, which will typically at least partially come in contact with the skin to the wearer and the garments of the wearer, is called the outer layer.

The layers of the bag material may be provided from any material, preferably so that the bag is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., Ill, US under the -designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a non-woven layer. Such material layers present an uneven surface to the skin of the wearer and thus reduce significantly the problem of occlusion and greatly improve skin healthiness.

In one preferred embodiment of the present invention the bag comprises two layers. Preferably the outer layer comprises a non-woven layer and the inner layer comprises a film.

In yet another preferred embodiment of the present invention, the bag (11) comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag (11) may be hydrophobic or hydrophilic. If the bag (11) does not comprise a film layer, preferably at least one non-woven layer is hydrophobic. As a consequence, fluid penetration is resisted through the wearer facing portion (16) and the garment facing portion (17) of the human waste management device (10). If the bag comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-ionic, cationic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

In one embodiment of the present invention the bag may contain absorbent material. The absorbent material may comprise any absorbent material which is capable of absorbing and retaining liquids. The absorbent material may Comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked celluiosic fibers; tissue, including tissue wraps and tissue laminates; absorbent roams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner layer of the bag (11). Any known techniques for securing absorbent material to nonwoven and film substrates may be used to secure the absorbent material to the inner layer of the bag. The absorbent material may also be arranged to have any desired shape or configuration (e.g., rectangular, oval, circular, etc.).

In the embodiment shown in Figure 4, the outer surface of bag (11) is provided with patches of adhesive (40) for securing the bag (11) to the body of the wearer. Preferably, the patches of adhesive (40) are positioned on the outer surface of bag (11) such that they are secured to the abdomen of the wearer in use. Any number, size and shape of adhesive patches (40) may be used depending on the intended use of the device.

The human waste management device in particular urine management devices according to the present invention also preferably comprise an additional acquisition layer. The acquisition layer is typically secured to the inner surface of bag. However, the acquisition layer may also be secured to the flange, or both the flange and the inner surface of bag. The acquisition layer is preferably positioned such that it separates the genitalia of the wearer from coming into direct contact with the absorbent material. The acquisition layer is fluid pervious allowing urine to readily pass through so that it may be absorbed by absorbent material.

The acquisition layer may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the acquisition, barrier layer includes fibers, the fibers may be spunbond, carded, wet-laid, meltblown, hydroentangled, or otherwise processed as is known in the art.

The acquisition layer is designed to have a pore size such that the absorbent material is not allowed to pass through and contact the wearer's skin. While designed not to have to large of a pore size which permits the passage of absorbent material, the acquisition layer preferably has a pore size which is greater than the pore size of the absorbent material.

Preferably, the acquisition layer is less hydrophilic than the absorbent material. The acquisition layer may be treated with a surfactant to increase its initial wettability. When treated with surfactant, however, the acquisition layer should still be less hydrophilic than the absorbent material. Suitable methods for treating the acquisition layer with a surfactant include spraying the acquisition layer with the surfactant and immersing the material into the surfactant. Alternatively, a surfactant may be incorporated into the acquisition layer.

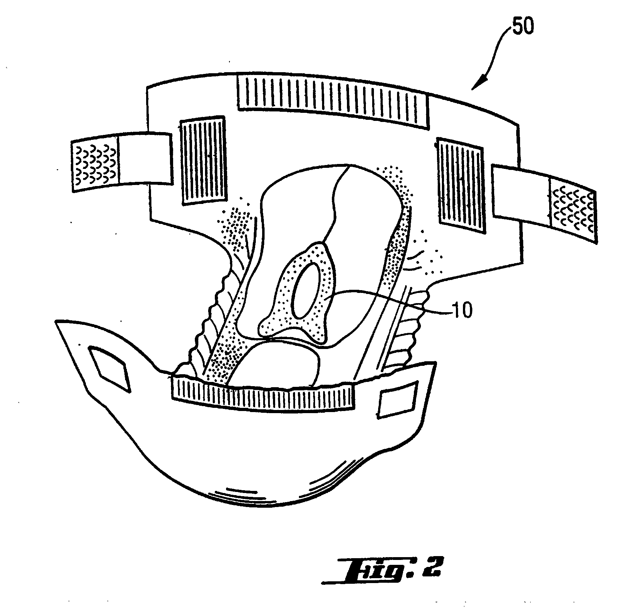

The human waste management device (10) of the present invention has been found to be particularly useful and beneficial when used in conjunction with a garment, or diaper (50), preferably a disposable diaper - refer to Figure 2. The device (10) is preferably first positioned in the perianal area of the wearer before the disposable diaper (50) is applied. In particular, the diaper (50) is positioned over the human waste management device (10) and fastened in a conventional manner around the body of the wearer. It has been found that, in addition, to providing excellent separation between urine and faecal material, the combined faecal management device (10) and diaper (50) system actually reduces skin irritation, which may at times occur, especially since the group of typical wearers includes the very old, the very young and the unhealthy wearers. In effect, the presence of the human waste management device (10) permits the formation of a separation layer between the skin of the wearer and the diaper (50), i.e. a part of the absorbent core (58) of the diaper (10). The diaper (50) can be of the conventional type (an embodiment of which is described below although not a limiting example by any means) or can be adapted to contain in an effective and comfortable manner the human waste management device (10) according to the teachings of the present invention.

As used herein, the term "disposable diapers" refers to articles which absorb and contain body extrudates; and more specifically, refers to articles which are placed against or in proximity to the body of the wearer to absorb and contain the various extrudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

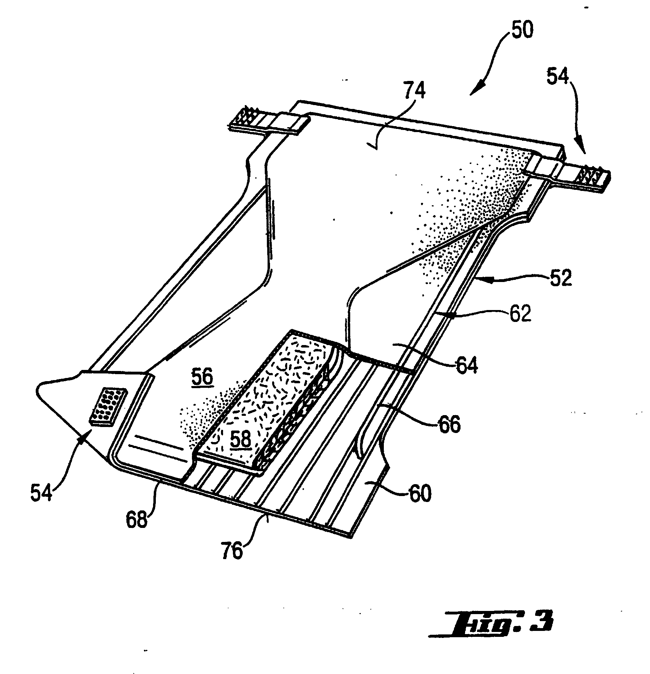

Figure 3 is a partially cut-away perspective view of a diaper (50) embodying the present invention prior to it being placed on the wearer over the faecal management device (10). As is visible from Figure 3, a preferred diaper (50) comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669. In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the diaper (50) opposite the outside surface (76) and in the embodiment shown is typically formed by the topsheet (56). In general, the inside surface (74) of the diaper (50) is that surface coextensive with the outside surface (76) and which is for the greater part in contact with the wearer when the diaper (50) is worn.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, melt-blown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the diaper (50) such as undergarments and bedding. The backsheet (60) may thus comprise polymeric films such as thermoplastic films of polyethylene or polypropylene, or composite materials such as film-coated non-woven material. Exemplary films are manufactured by Tredegar Industries, Inc. of Terre Haute, Ind., USA or BP-Chemical PlasTec, itlotbuchenstrasse 1, D-8000 München, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a non-woven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

**Test methods.**

Air and Vapour Permeability Test

The Vapour permeability test is utilised to quantify the vapour transmission properties of the skin attachment means of faecal management-devices.

**Basic Principle of the Methods:**

The basic principle of the test is to quantify the extent of water vapour transmission through the skin attachment means of the faecal management device. The test method that is applied is based on a standardized textile industry applied test method and commonly referred to as the "cup test method". The test is performed in a stable temperature/humidity laboratory maintained at a temperature of 23° C at 50% RH for a period of 24 hours.

**Apparatus:**

    1) Sample cup of dimensions having an open area = 0.003 m$^2$)
    2) Syringe to introduce the distilled water into the completed sample cup.
    3) Wax to seal the cup once sample has been arranged.
    4) A circular punch to facilitate preparation circular samples of diameter = 30 mm.
    5) Laboratory of stable climatic conditions (23° C $\pm$ 0.5°C / 50% RH $\pm$ 1 % RH)
    6) Laboratory balance accurate to 4 decimal places.

**Sample Preparation / Measurements:**

The test is to be performed on the skin attachment means of the device. A representative device is selected and a sample is cut to size using the punch. The sample cut is sufficiently large to adequately overlap the sample holder and to ensure material that may have been damaged or undesirably stretched due to the cutting operation lies outside of the measurement centre when the measurement is performed. The sample is so arranged onto of the sample cup so as to fully overlap the cup. The sample is oriented so as to ensure that the surface exposed to the laboratory environment is the same that would be found while wearing the article.

The closure ring of the sample cup is then placed onto the sample and pushed down. This ensures that the excess material is held firmly in place and does not interfere with the measurement. A wax is then applied to the entire surface of the closure ring to ensure the whole upper part of the apparatus is closed to the environment. Distilled water $(5 \pm 0.25 \text{ ml})$ is introduced with the syringe into the sealed sample cup via the minute perforation. Finally this perforation is sealed with silicone grease.

The entire cup (containing sample and water) is weighed and the weight recorded to 4 decimal places. The cup is then placed in a ventilation stream generated by a fan. The air flowing over the top of the sample cup is $3 \pm 0.3$ m/sec and confirmed via a wind velocity meter ("Anemo", supplied by Deuta SpA., Italy). The sample cup remains in the ventilated test field for a period of 24 hrs and is then re-weighted. During this period if the test sample is sufficiently breathable the liquid in the sample holder is able to diffuse out of the sample holder and into the laboratory environment. This results in a reduction in the weight of water in the sample holder that can be quantified on re-weighing the complete sample cup following the 24 hr period.

The vapour permeability value is determined as the weight loss divided by the open area of the sample holder and quoted per day.

$$\text{i.e. Vapour Permeability} = \text{Weight Loss (g)} / (0.003 \text{ m}^2 / 24 \text{ hrs})$$

**Air Permeability test:**

The air permeability test is utilised to assess the ability of skin attachment means of the faecal management devices to circulationlexchange air.

**Basic Principle of the Methods:**

The basic principle of the test is to evaluate the resistance of the skin attachment means to the passage of air. In this test, the volume (or amount) of air that flows through an article of given dimensions under standard conditions (of 23 °C /50% RH) is measured. The instrument utilised for the test is: Air Permeabilimeter FX 3300 manufactured by TexTest AG Switzerland.

Samples should be allowed to equilibrate in the test environment for at least 4 hrs prior to commencement of the measurement. The article (having dimensions exceeding 5 $\text{cm}^2$ the dimensions of the measurement head) is placed on the device as instructed by the manufacturer. An aspiration pump set to generate a pressure of 1210 kPa that sucks air through the sample layer or structure. The device measures the volume of air flow and the pressure drop across the orifices that contains the sample and measurement head. Finally the device generates a value of air permeability in the units of "I/M$^2$s$^1$".

**Claims**

1. A human waste management device (10) comprising a bag (11), said bag (11) having an aperture (21) and said bag (11) having a skin attachment means surrounding said aperture (21), said skin attachment means comprising a flange (21) having a wearer facing surface (23) and a garment facing surface (22), and wherein said wearer facing surface (23) of said flange (12) comprises an adhesive (20) **characterised in that** said skin attachment means is moisture vapour permeable and has a moisture vapour transport rate of at least 250g/(m$^2$.24hrs.).

2. A human waste management device (10) according to claim 1 wherein said skin attachment means has a moisture vapour transport rate of at least 300 g/(m$^2$.24hrs.).

3. A human waste management device (10) according to claim 2 wherein said skin attachment means has a air permeability of at least 50 I(m$^2$s).

**EP 1 089 695 B1**

4. A human waste management device according to any one of the preceding claims, wherein said flange comprises a material selected from wovens, nonwovens, open celled foams, monolithic films, microporous films, macroporous films, apertured formed films and any combination thereof.

5. A human waste management device according to claim 4, wherein said flange is selected from open celled foams, nonwovens or a combination thereof.

6. A human waste management device (10) according to any one of the preceding claims wherein said adhesive (20) is a moisture vapour permeable medically approved pressure sensitive adhesive comprising a polymer which forms a 3-dimensional matrix by being crosslinked, and comprises less than 10% by weight of a hydrocolloid.

7. A human waste management device according to claim 6, wherein said adhesive is hydrophilic.

8. A human waste management device (10) according to claim 7, wherein said adhesive is applied to said wearer facing surface (23) of said flange (12) at a basis weight of from 20 g/m$^2$ to 2500g/m$^2$.

9. The use of a human waste management device (10) according to any of the preceding claims in combination with a disposable diaper (50).

10. The use of a human waste management device (10) according to claim 9 whereby said faecal management device (10) is first positioned in the perianal area of the wearer and then said disposable diaper (50) is positioned over said faecal management device (10) and fastened in a conventional manner around body of said wearer.


**Patentansprüche**

1. Managementvorrichtung (10) für menschliche Ausscheidungen mit einer Tasche (11), wobei die Tasche (11) eine Öffnung (21) und die Tasche (11) ein die Öffnung (21) umgebendes Hautanbringungsmittel aufweist, wobei das Hautanbringungsmittel einen Flansch (21) umfaßt, der eine trägerseitige Oberfläche (23) und eine wäscheseitige Oberfläche (22) aufweist und in welcher die trägerseitige Oberfläche (23) des Flansches (12) ein Haftmittel (20) umfaßt, **dadurch gekennzeichnet, daß** das Hautanbringungsmittel wasserdampfdurchlässig ist und eine Wasserdampf-Transportgeschwindigkeit von wenigstens 250 g/(m$^2$ 24 h) hat.

2. Managementvorrichtung (10) für menschliche Ausscheidungen gemäß Anspruch 1, in welcher das Hautanbringungsmittel eine Wasserdampf-Transportgeschwindigkeit von wenigstens 300 g/(m$^2$ 24 h) hat.

3. Managementvorrichtung (10) für menschliche Ausscheidungen gemäß Anspruch 2, in welcher das Hautanbringungemittel eine Luftdurchlässigkeit von wenigstens 50 1 (m$^2$s) hat.

4. Managementvorrichtung für menschliche Ausscheidungen gemäß einem der vorstehenden Ansprüche, in welcher der Flansch ein Material umfaßt, ausgewählt aus Geweben, Vliesstoffen, offenzelligen Schäumen, monolithischen Filmen, mikroporösen Filmen, makroporösen Filmen, offen geformten Filmen und einer Kombination davon.

5. Managementvorrichtung für menschliche Ausscheidungen gemäß Anspruch 4, in welcher der Flansch ausgewählt ist aus offenzelligen Schäumen, Vliesstoffen oder Kombinationen davon.

6. Managementvorrichtung (10) für menschliche Ausscheidungen gemäß einem der vorstehenden Ansprüche, in welcher das Haftmittel (20) ein wasserdampfdurchlässiges medizinisch zugelassenes, druckempfindliches Haftmittel ist, das ein Polymer aufweist, welches durch Vernetzung eine dreidimensionale Matrix bildet und weniger als 10 Gew.% eines Hydrokolloids umfaßt.

7. Managementvorrichtung für menschliche Ausscheidungen gemäß Anspruch 6, in welcher das Haftmittel hydrophil ist.

8. Managementvorrichtung (10) für menschliche Ausscheidungen gemäß Anspruch 7, in welcher das Haftmittel auf der trägerseitigen Oberfläche (23) des Flansches (12) mit einem Basisgewicht von 20 g/m$^2$ bis 2500 g/m$^2$ aufgebracht ist.

**9.** Verwendung einer Managementvorrichtung (10) für menschliche Ausscheidungen gemäß einem der vorstehenden Ansprüche in Kombination mit einer Einwegwindel (50).

**10.** Verwendung einer Managementvorrichtung (10) für menschliche Ausscheidungen gemäß Anspruch 9, bei welcher die Stuhlgang-Managementvorrichtung (10) zuerst in dem perianalen Gebiet des Trägers positioniert wird und dann die Einwegwindel (50) über der Stuhlgang-Managementvorrichtung (10) positioniert und in einer herkömmlichen Weise um den Körper des Trägers herum befestigt wird.

**Revendications**

**1.** Dispositif de gestion de déchets humains (10), comprenant un sac (11), ledit sac (11) comportant une ouverture (21) et ledit sac (11) comportant un moyen de fixation à la peau entourant ladite ouverture (21), ledit moyen de fixation à la peau comprenant un rebord (12) présentant une surface (23) faisant face à l'utilisateur et une partie (22) faisant face au vêtement, et dans lequel ladite surface (23) faisant face à l'utilisateur dudit rebord (12) comprend un adhésif (20), **caractérisé en ce que** ledit moyen de fixation à la peau est perméable à la vapeur et a une vitesse de transport de vapeur au moins de 250 g/(m$^2$.24 h).

**2.** Dispositif de gestion de déchets humains (10) selon la revendication 1, dans lequel ledit moyen de fixation à la peau a une vitesse de transport de vapeur au moins de 300 g/(m$^2$.24 h).

**3.** Dispositif de gestion de déchets humains (10) selon la revendication 2, dans lequel ledit moyen de fixation à la peau a une perméabilité à l'air au moins de 50 l(m$^2$s).

**4.** Dispositif de gestion de déchets humains selon l'une quelconque des revendications précédentes, dans lequel ledit rebord comprend un matériau choisi parmi les tissés, les non-tissés, les mousses à alvéoles ouvertes, les films monolithiques, les films microporeux, les films macroporeux, les films formés perforés et n'importe laquelle de leurs combinaisons.

**5.** Dispositif de gestion de déchets humains selon la revendication 4, dans lequel ledit rebord est choisi parmi les mousses à alvéoles ouvertes, les non tissés, ou une de leurs combinaisons.

**6.** Dispositif de gestion de déchets humains (10) selon l'une quelconque des revendications précédentes, dans lequel ledit adhésif (20) est un adhésif sensiblement à la pression, approuvé médicalement, perméable à la vapeur, comprenant un polymère qui forme une matrice à trois dimensions en étant réticulé, et comprend moins de 10% en poids d'un hydrocolloïde.

**7.** Dispositif de gestion de déchets humains selon la revendication 6, dans lequel ledit adhésif est hydrophile.

**8.** Dispositif de gestion de déchets humains (10) selon la revendication 7, dans lequel ledit adhésif est appliqué à ladite surface (23) faisant face à l'utilisateur dudit rebord (12) pour un grammage de 20 g/m$^2$ à 2500 g/m$^2$.

**9.** Utilisation d'un dispositif de gestion de déchets humains (10) selon l'une quelconque des revendications précédentes en combinaison avec une couche jetable (50).

**10.** Utilisation d'un dispositif de gestion de déchets humains (10) selon la revendication 9, dans lequel ledit dispositif de gestion de déchets humains (10) est placé, d'abord, dans la zone périanale de l'utilisateur et, ensuite, ladite couche jetable (50) est placée sur ledit dispositif de gestion de déchets humains (10) et fixée de manière classique autour du corps dudit utilisateur.

Fig. 1

EP 1 089 695 B1

**Fig: 2**

Fig. 3

## Fig. 4